# FASCICULE DE BREVET EUROPEEN

(11) **EP 3 225 613 B1**
(45) Date de publication et mention de la délivrance du brevet: **19.09.2018**
(21) Numéro de dépôt: 17156083.2
(22) Date de dépôt: 14.02.2017
(51) Int. Cl.: C07C 51/44, C07C 51/25, C07C 51/42, C07C 57/04, G09F 9/30, G09F 21/04, G09F 7/18, H05K 5/02

(54) **PROCEDE AMELIORE DE PRODUCTION D'ACIDE (METH)ACRYLIQUE**
VERBESSERTES VERFAHREN ZUR HERSTELLUNG VON (METH)ACRYLSÄURE
IMPROVED METHOD FOR PRODUCING (METH)ACRYLIC ACID

(30) Priorité: 29.03.2016 FR 1652683
(43) Date de publication de la demande: 04.10.2017
(73) Titulaire: Arkema France, 92700 Colombes (FR)
(72) Inventeur: JAIN, Sandeep, 75003 Paris (FR); LACROIX, Christian, 57600 Forbach (FR)

(56) Documents cités:
- WO-A2-2008/033687

## Description

### DOMAINE TECHNIQUE

La présente invention concerne la production d'acide (méth)acrylique.

Elle a plus particulièrement pour objet la mise en oeuvre d'une étape de condensation de l'eau contenue dans un effluent gazeux recyclé et/ou dans l'alimentation en air, dans un procédé de production d'acide (méth)acrylique qui inclut un procédé de purification d'un mélange réactionnel comprenant de l'acide (méth)acrylique sans utiliser de solvant azéotropique et basé sur la mise en oeuvre de deux colonnes de distillation.

Le procédé selon l'invention permet de réduire les pertes en acide (méth)acrylique au cours de la purification, et globalement d'améliorer l'efficacité du procédé.

### ARRIERE-PLAN TECHNIQUE ET PROBLEME TECHNIQUE

Le procédé de synthèse d'acide acrylique exploité à grande échelle industrielle, met en oeuvre une réaction d'oxydation catalytique du propylène en présence d'oxygène.

Cette réaction est conduite généralement en phase gazeuse, et le plus souvent en deux étapes : la première étape réalise l'oxydation sensiblement quantitative du propylène en un mélange riche en acroléine, puis, lors de la deuxième étape réalise l'oxydation sélective de l'acroléine en acide acrylique.

Le mélange gazeux issu de la deuxième étape est constitué, en dehors de l'acide acrylique :
- des impuretés issues de la première étape de réaction qui n'ont pas réagi (propylène, propane);
- de composés légers incondensables dans les conditions de température et de pression habituellement mises en oeuvre, non transformés dans la première étape ou formés dans la seconde étape : azote, oxygène non converti, monoxyde et dioxyde de carbone formés en faible quantité par oxydation ultime ou tournant en rond, par recyclage, dans le procédé ;
- de composés légers condensables non transformés dans la première étape ou formés dans la seconde étape : l'eau, l'acroléine non converti, des aldéhydes légers comme le formaldéhyde et l'acétaldéhyde, l'acide formique, l'acide acétique ou l'acide propionique ;
- de composés lourds : furfuraldéhyde, benzaldéhyde, acide et anhydride maléique, acide benzoïque, acide 2-buténoïque, phénol, protoanémonine.

La complexité du mélange gazeux obtenu dans ce procédé nécessite de procéder à un ensemble d'opérations pour récupérer l'acide acrylique contenu dans cet effluent gazeux et le transformer en un grade d'acide acrylique compatible avec son utilisation finale, par exemple la synthèse d'esters acryliques ou la production de polymères d'acide acrylique et/ou d'esters acryliques.

Une nouvelle technologie de récupération/purification d'acide acrylique est apparue récemment, impliquant un nombre réduit d'étapes de purification et ne nécessitant pas de solvant organique externe.

Le brevet EP 2 066 613, basé sur cette technologie « sans solvant », décrit un procédé de récupération d'acide acrylique sans utiliser d'eau extérieure, ni de solvant azéotropique et ne mettant en oeuvre que deux colonnes de purification du mélange réactionnel gazeux refroidi : a) une colonne de déshydratation, b) et une colonne de finition (ou colonne de purification) alimentée par une partie du flux de pied de la colonne de déshydratation.

Selon ce procédé, le flux réactionnel gazeux refroidi est soumis à une déshydratation dans une première colonne. Le flux gazeux distillé en tête de colonne est envoyé dans un condenseur, dans lequel les composés légers sont en partie condensés et renvoyés à la colonne de déshydratation sous forme de reflux pour absorber l'acide acrylique, l'effluent gazeux étant renvoyé au moins en partie vers la réaction et le reste étant incinéré.

Le flux de pied de la colonne de déshydratation alimente une seconde colonne qui permet de séparer par soutirage latéral, sous forme de liquide ou de vapeur, un flux d'acide acrylique purifié correspondant à un grade technique. L'acide acrylique technique obtenu est généralement de pureté supérieure à 98,5% massique et contient moins de 0,5% massique d'eau.

Dans cette colonne de finition, le distillat de tête comprenant de l'eau et des sous-produits légers est condensé puis recyclé en pied de la première colonne, et un flux comprenant de l'acide acrylique enrichi en sous-produits lourds est éliminé en pied pour être utilisé éventuellement pour la production d'esters acryliques.

Dans ce procédé, une partie des flux (du pied de la colonne de déshydratation ou de tête de la colonne de finition) est renvoyée avantageusement aux dispositifs de chauffage/rebouilleur de la colonne de déshydratation et/ou utilisée pour refroidir le mélange réactionnel gazeux, ce qui permet d'optimiser les besoins énergétiques du procédé.

Malgré les avantages que procure le procédé de purification décrit dans le document EP 2 066 613, il subsiste encore des inconvénients liés à sa mise en oeuvre, notamment au niveau de la perte possible d'acide acrylique durant les différentes étapes.

En particulier, de l'acide acrylique peut être entraîné en tête de la colonne de déshydratation. Selon l'équilibre liquide/vapeur à la température de fonctionnement du condenseur placé en tête, l'effluent gazeux à la sortie peut contenir de l'acide acrylique en quantité non négligeable. L'acide acrylique est directement perdu dans la partie de l'effluent gazeux qui est incinérée.

Il subsiste donc un besoin de réduire les pertes en acide acrylique dans un procédé de récupération/purification sans solvant basé sur la mise en oeuvre d'une colonne de déshydratation et d'une colonne de finition, et en particulier de réduire les pertes en acide acrylique en tête de la colonne de déshydratation.

Les inventeurs ont maintenant découvert que la perte en acide acrylique peut être réduite en contrôlant la teneur en eau introduite dans ce procédé.

La principale source d'eau provient du mélange réactionnel brut à traiter, puisqu'il contient l'eau formée au cours de la réaction d'oxydation catalytique du propylène en acide acrylique. L'eau est la principale impureté « légère » qui est distillée en tête de la colonne de déshydratation et son élimination au cours du procédé de purification conditionne la qualité recherchée de l'acide acrylique purifié (teneur en eau < 0,5% massique).

Il est nécessaire d'éliminer l'eau de façon optimale en tête de la colonne de déshydratation tout en minimisant la perte en acide acrylique. Il est possible par exemple d'abaisser la température du condenseur placé en tête, pour modifier l'équilibre liquide/vapeur et éviter l'entrainement d'acide acrylique dans l'effluent gazeux. Cela n'est cependant possible que dans une certaine limite dépendante de la teneur en eau introduite dans la colonne de déshydratation.

Outre l'eau inhérente au procédé de synthèse de l'acide acrylique, de l'eau est introduite par d'autres voies, telles que par exemple l'humidité présente dans le flux d'air introduit à la réaction pour réaliser l'oxydation du propylène. Par ailleurs, des solutions aqueuses d'inhibiteurs de polymérisation sont introduites au niveau des colonnes et/ou des condenseurs pour limiter les réactions de polymérisation, et l'eau ainsi introduite peut tourner en rond dans le procédé par recyclage et/ou reflux.

Il a été découvert qu'en réduisant ces sources annexes d'eau, l'élimination de l'eau en tête de la colonne de déshydratation peut être optimisée, tout en minimisant les pertes en acide acrylique.

Selon l'invention, il est proposé de condenser l'eau présente dans le flux d'air alimentant la réaction, et/ou l'eau présente dans l'effluent gazeux issu de la colonne de déshydratation qui est recyclé à la réaction.

Il est par ailleurs apparu aux inventeurs que cette invention pouvait s'appliquer à l'acide acrylique produit à partir d'autres sources que le propylène, à l'acide méthacrylique, ainsi qu'à ces acides dérivés de matières premières renouvelables, qui sont susceptibles de poser les mêmes problèmes de purification liés à la présence d'eau.

### RESUME DE L'INVENTION

La présente invention concerne en premier lieu un procédé de production d'acide (méth)acrylique, comprenant au moins les étapes suivantes :
i) on soumet à une oxydation en phase gazeuse en présence d'air, au moins un précurseur d'acide (méth)acrylique pour former un mélange réactionnel gazeux comprenant de l'acide (méth)acrylique ;
ii) on refroidit le mélange réactionnel gazeux ;
iii) on soumet le mélange réactionnel gazeux à une déshydratation sans utiliser de solvant azéotropique dans une première colonne dite colonne de déshydratation, conduisant à un flux gazeux de tête et à un flux de pied ;
iv) on soumet le flux gazeux distillé en tête de colonne de déshydratation, au moins en partie, à une condensation dans un condenseur de tête, le condensat étant renvoyé à la colonne de déshydratation sous forme de reflux pour absorber l'acide acrylique, et l'effluent gazeux étant renvoyé au moins en partie vers la réaction d'oxydation et le reste étant soumis à une oxydation thermique et/ou catalytique ;
v) on soumet au moins en partie le flux de pied de colonne de déshydratation à une distillation dans une seconde colonne dite colonne de finition, conduisant à un flux de tête, et à un flux de pied contenant des composés lourds ;
vi) on récupère un flux d'acide (méth)acrylique par soutirage latéral de la colonne de finition ;
ledit procédé étant caractérisé en ce que l'on met en oeuvre une étape de condensation de l'eau appliquée à l'un au moins des deux flux suivants : l'alimentation en air pour la réaction d'oxydation de l'étape i), ou l'effluent gazeux à la sortie du condenseur de tête de l'étape iv) qui est recyclé à la réaction d'oxydation.

Dans la présente invention, le terme « (méth)acrylique » signifie « acrylique » ou « méthacrylique ».

Le terme « solvant azéotropique » désigne tout solvant organique présentant la propriété de former un mélange azéotropique avec l'eau.

Le terme « léger » qualifiant les composés sous-produits désigne les composés dont le point d'ébullition est inférieur à celui de l'acide (méth)acrylique, et par analogie, le terme « lourd » désigne les composés dont le point d'ébullition est supérieur à celui de l'acide (méth)acrylique.

Le procédé selon l'invention peut comprendre en outre d'autres étapes visant à poursuivre la purification du flux d'acide (méth)acrylique récupéré à l'étape vi).

Selon un mode réalisation de l'invention, le précurseur de l'acide (méth)acrylique est l'acroléine.

Selon un mode réalisation de l'invention, l'acroléine est obtenue par oxydation de propylène ou par oxydéshydrogénation de propane.

Selon un mode réalisation de l'invention, le précurseur de l'acide (méth)acrylique est la méthacroléine.

Selon un mode réalisation de l'invention, la méthacroléine est obtenue par oxydation d'isobutylène et/ou de tert-butanol.

Selon un mode réalisation de l'invention, la méthacroléine est obtenue à partir d'oxydéshydrogénation de butane et/ou isobutane.

Selon un mode réalisation de l'invention, le précurseur de l'acide (méth)acrylique est dérivé du glycérol, de l'acide 3-hydroxypropionique ou de l'acide 2-hydroxypropanoïque (acide lactique).

Selon un mode réalisation préféré de l'invention, l'acide (méth)acrylique est l'acide acrylique et le précurseur de l'acide acrylique est l'acroléine obtenu par oxydation catalytique de propylène. Le mélange réactionnel gazeux comprend de l'acide acrylique dérivé de propylène obtenu selon un procédé d'oxydation en deux étapes.

Selon certains modes de réalisation particuliers, l'invention présente également une ou, de préférence, plusieurs des caractéristiques avantageuses énumérées ci-dessous :
- l'étape de condensation de l'eau est réalisée à l'aide d'un condenseur unique pour les deux flux, ou à l'aide d'un condenseur pour chacun des deux flux ;
- l'étape de condensation de l'eau est réalisée à une température allant de 15°C à la température du condenseur de tête de la colonne de déshydratation ;
- l'étape de condensation de l'eau pour l'effluent gazeux recyclé est réalisée de préférence à une température comprise entre 40°C et 60°C, plus préférentiellement entre 45°C et 55°C ;
- l'étape de condensation de l'eau pour l'alimentation en air est réalisée de préférence à une température comprise entre 15°C et 25°C ;
- l'eau condensée est utilisée en partie pour préparer des solutions aqueuses d'inhibiteur de polymérisation qui sont introduites dans la colonne de déshydratation et/ou dans la colonne de finition, ou leurs condenseurs associés ;
- l'eau condensée est en partie purgée et envoyée au traitement des eaux usées, ou soumise à un traitement d'oxydation thermique.

Le procédé selon l'invention permet de réduire la formation d'une boucle d'eau entre la réaction et la colonne de déshydratation, l'eau condensée étant avantageusement utilisée pour préparer les solutions aqueuses d'inhibiteur de polymérisation, nécessaires pour le bon fonctionnement du procédé. L'introduction d'eau externe pour préparer les solutions d'inhibiteurs est donc limitée et l'acide acrylique éventuellement présent dans l'eau condensée est recyclé directement dans le train de purification, au lieu d'être renvoyé dans le réacteur où il peut être oxydé de façon ultime en monoxyde ou dioxyde de carbone.

L'absence d'eau externe permet d'adapter les conditions de fonctionnement du condenseur de tête de la colonne de déshydratation en réduisant sa température, et en conséquence les pertes en acide acrylique sont limitées.

En outre, les performances du catalyseur d'oxydation du précurseur d'acide (méth)acrylique et sa durée de vie peuvent être améliorées du fait d'une teneur en eau et en impuretés organiques plus faible dans le réacteur d'oxydation.

Le procédé selon l'invention peut contenir en outre d'autres étapes ou d'autres caractéristiques pour autant qu'elles n'affectent pas négativement l'effet procuré par la présente invention.

D'autres caractéristiques et avantages de l'invention ressortiront mieux à la lecture de la description détaillée qui suit, en référence aux figures 1 à 5 annexées qui représentent :
- Figure 1 : installation adaptée pour mettre en oeuvre le procédé selon l'invention
- Figure 2 : schéma de l'art antérieur
- Figure 3 : schéma illustrant un premier mode de réalisation de l'invention.
- Figure 4 : schéma illustrant un second mode de réalisation de l'invention
- Figure 5 : schéma illustrant un troisième mode de réalisation de l'invention.

### DESCRIPTION DETAILLEE DE L'INVENTION

L'invention est basée sur l'intégration d'au moins un condenseur dans un procédé de production d'acide (méth)acrylique incluant un procédé de récupération/purification sans solvant de l'art antérieur.

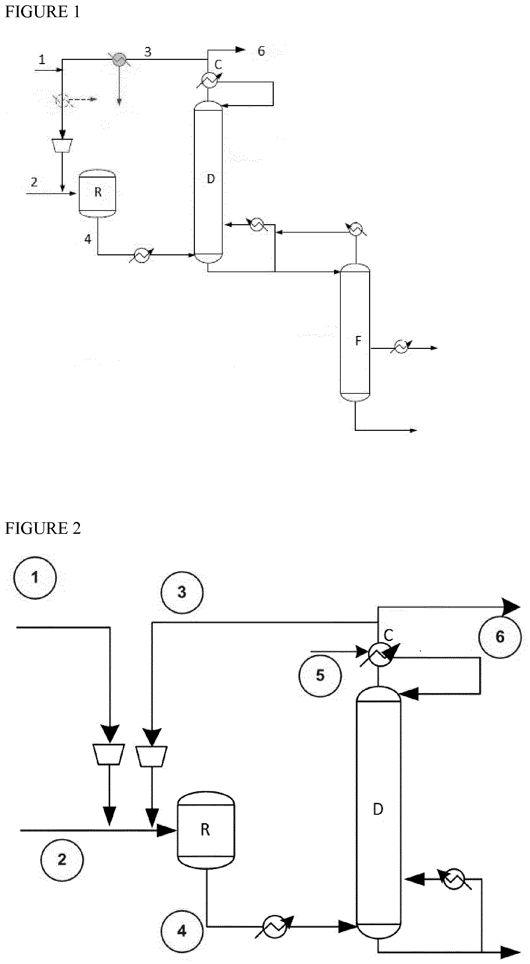

Sur la Figure 1, est représenté un réacteur R produisant un mélange réactionnel gazeux 4 comprenant de l'acide (méth)acrylique obtenu par oxydation en phase gazeuse avec de l'air 1 d'un précurseur de l'acide (méth)acrylique 2.

Selon un mode de réalisation, le réacteur R est un ensemble de 2 réacteurs en série ou comprend au moins 2 zones réactionnelles en série, le premier réacteur ou la première zone de réaction étant utilisée pour la synthèse du précurseur de l'acide (méth)acrylique.

Le mélange réactionnel gazeux comportant un rapport massique eau/acide (méth)acrylique généralement compris entre 0,3 et 2 peut être préalablement refroidi avant d'être envoyé dans une première colonne dite colonne de déshydratation D.

La colonne de déshydratation comporte en tête un condenseur de tête C dans lequel les composés légers et l'eau sont en partie condensés et renvoyés à la colonne de déshydratation sous forme de reflux pour absorber l'acide acrylique. L'effluent gazeux, comprenant les composés légers incondensables et de l'eau, est renvoyé au moins en partie vers la réaction (flux 3) et le reste (flux 6) est envoyé à un dispositif d'épuration, à un oxydeur thermique et/ou un oxydeur catalytique, ou est incinéré.

Selon un mode de réalisation, la totalité du flux de tête de la colonne de déshydratation est envoyée dans le condenseur de tête.

La colonne de déshydratation D fonctionne, au moins partiellement, comme une colonne de distillation, généralement à la pression atmosphérique ou légèrement supérieure, jusque 1,5 10⁵ Pa. Avantageusement, la température dans la partie supérieure de la colonne de déshydratation est d'au moins 40°C, de préférence est comprise entre 40°C et 80°C. La température du flux de pied de la colonne de déshydratation ne dépasse pas de préférence 120°C.

Aucun solvant azéotropique n'est ajouté dans la colonne de déshydratation.

La colonne de déshydratation génère un flux de pied comprenant l'essentiel de l'acide (méth)acrylique avec des sous-produits lourds et une teneur massique en eau généralement inférieure à 10%, de préférence inférieure à 7%.

Le flux de pied de la colonne de déshydratation est envoyé au moins en partie en tête d'une seconde colonne de distillation, dite colonne de finition F, ou colonne de purification.

La colonne de déshydratation et la colonne de finition peuvent être de configurations diverses, par exemple de type colonne à garnissage vrac ou structuré ou colonnes à plateaux.

La température et la pression dans la colonne de purification ne sont pas critiques, et peuvent être déterminées conformément aux méthodes de distillation connues de l'état de l'art. Cependant, de préférence, la colonne de purification fonctionne à une pression inférieure à la pression atmosphérique, permettant de fonctionner à des températures relativement faibles, évitant ainsi la polymérisation des produits insaturés présents, et minimisant la formation de sous-produits lourds.

Avantageusement, la colonne de purification fonctionne sous une pression allant de 5 kPa à environ 60 kPa, la température du flux de tête étant avantageusement comprise entre 40°C et environ 90°C, et la température du flux de pied étant comprise ente 60°C et 120°C.

La colonne de finition génère un distillat de tête comprenant de l'eau et des sous-produits légers, qui est condensé puis recyclé en pied de la première colonne, et un flux de pied comprenant de l'acide acrylique enrichi en sous-produits lourds qui est éliminé en pied pour être utilisé éventuellement pour la production d'esters acryliques.

Le flux soutiré latéralement de la colonne de finition F correspond à un grade d'acide (méth)acrylique technique. Il est en général constitué d'acide (méth)acrylique de pureté supérieure à 98%, de préférence supérieure à 99%. De préférence, il contient moins de 1,5%, de préférence moins de 0,5%, plus particulièrement moins de 0,2% en poids d'acide acétique, et moins de 1%, de préférence moins de 0,5%, plus particulièrement, moins de 0,3% en poids d'eau. Ce flux peut encore être soumis à une purification par distillation, éventuellement couplée avec un traitement de cristallisation.

Selon le procédé de l'invention, au moins un condenseur est placé sur l'effluent gazeux 3 qui est recyclé dans le réacteur R et/ou sur l'alimentation en air 1, afin de condenser au moins l'eau présente dans ces flux.

On peut utiliser un seul condenseur, ou deux condenseurs comme représenté sur la figure 1.

De l'eau réfrigérée ou de l'eau froide est utilisée pour condenser le gaz. La température de l'eau peut aller d'environ 8°C à environ 45°C selon la température de condensation. Le condenseur placé sur l'alimentation de l'air est préférablement refroidi avec de l'eau réfrigérée à environ 8°C. Le condenseur placé sur l'effluent gazeux recyclé est de préférence refroidi avec de l'eau à température ambiante (de l'ordre de 25°C).

Le condenseur peut être de configurations diverses, tel qu'un échangeur à faisceau tubulaire, un échangeur à spirales, un échangeur à tubes à ailettes, ou un condenseur à contact liquide, etc.

La température de condensation de l'eau peut aller de 15°C à la température du condenseur de tête de la colonne de déshydratation qui est généralement inférieure à 65°C.

Selon un mode de réalisation, la température du condenseur placé au niveau de l'effluent gazeux 3 est comprise entre 40°C et 60°C, de préférence entre 45°C et 55°C.

Selon un mode de réalisation, la température du condenseur placé au niveau de l'alimentation en air 1, est comprise entre 15°C et 25°C.

Selon un mode de réalisation, l'eau condensée issue de l'alimentation en air, qui est exempte d'impuretés organiques, est avantageusement recyclée au moins en partie dans les tours de refroidissement d'eau.

Selon un mode de réalisation, l'eau condensée est utilisée en partie pour préparer des solutions aqueuses d'inhibiteur de polymérisation qui peuvent être introduites dans l'installation à différents endroits.

Les inhibiteurs de polymérisation sont choisis parmi les composés qui inhibent la réaction de polymérisation de l'acide (méth)acrylique Comme exemples de composés utilisables, on peut citer la phénothiazine, l'hydroquinone, le 2,2,6,6-tétraméthyl-1-pipéridinyloxy (Tempo) ou l'un de ses dérivés tel que le 4-hydroxy Tempo, les sels de cuivre solubles, les sels de manganèse solubles, seuls ou en mélange.

Les solutions aqueuses d'inhibiteur de polymérisation sont ajoutées en quantité suffisante connue de l'homme de l'art pour éviter ou réduire la polymérisation de l'acide (méth)acrylique dans l'installation, notamment dans le flux de tête de la colonne de déshydratation au niveau du condenseur de tête, ou dans le flux de tête de la colonne de finition, au niveau du condenseur associé à ladite colonne, ou dans le flux de produit purifié soutiré latéralement de la colonne de finition, éventuellement après condensation dans le cas où le flux soutiré est sous forme gazeuse.

Le procédé selon l'invention conduit à la production d'acide (méth)acrylique avec un rendement amélioré par rapport aux procédés de l'art antérieur. En effet, l'utilisation d'un condenseur sur l'effluent gazeux recyclé à la réaction et/ou à l'alimentation de l'air a permis de réduire la perte en acide acrylique de plus de 50% par rapport à un procédé qui ne comporte pas d'étape de condensation afin de limiter l'entrée d'eau dans le réacteur. Par ailleurs, outre la réduction des pertes en acide acrylique, il a été possible d'abaisser la température du condenseur de tête de l'ordre de 2 à 4°C, ce qui présente un avantage supplémentaire au niveau énergétique.

L'invention sera maintenant illustrée par les exemples suivants, qui n'ont pas pour but de limiter la portée de l'invention, définie par les revendications annexées.

### PARTIE EXPERIMENTALE

### Exemple 1 (référence)

En référence à la figure 2, représentant un procédé de l'art antérieur pour produire de l'acide acrylique (AA), un réacteur est alimenté par un flux 2 de propylène et un flux 1 d'air.
A la sortie du réacteur, un mélange réactionnel gazeux comportant l'acide acrylique produit, est envoyé après refroidissement dans un échangeur, à une colonne de déshydratation D surmontée d'un condenseur de tête C. Un flux 5 d'une solution d'inhibiteur aqueuse d'inhibiteur de polymérisation est introduite au niveau du condenseur de tête C. Une partie de l'effluent gazeux 3 est recyclé dans le réacteur, et une partie de l'effluent gazeux 6 est envoyée vers un incinérateur.
Une simulation à l'aide du logiciel Aspen a été utilisée pour caractériser la perte en acide acrylique dans ce type d'installation.

Les résultats sont présentés dans le tableau 1 ci-après :

**Tableau 1**

| | |
|---|---|
| AA produit par la réaction, kg/h | 11805 |
| AA perdu en tête du condenseur C, kg/h | 206 |
| Perte d'AA en tête de C, % | 1,74 |
| Température du condenseur de tête C, °C | 60,9 |
| Eau en entrée du réacteur R, % | 6,58 |
| Eau en sortie du réacteur R, kg/h | 6660 |
| AA en sortie du réacteur R, kg/h | 11871 |
| Rapport AA/eau en sortie du réacteur R | 1,78 |

### Exemple 2 (selon l'invention)

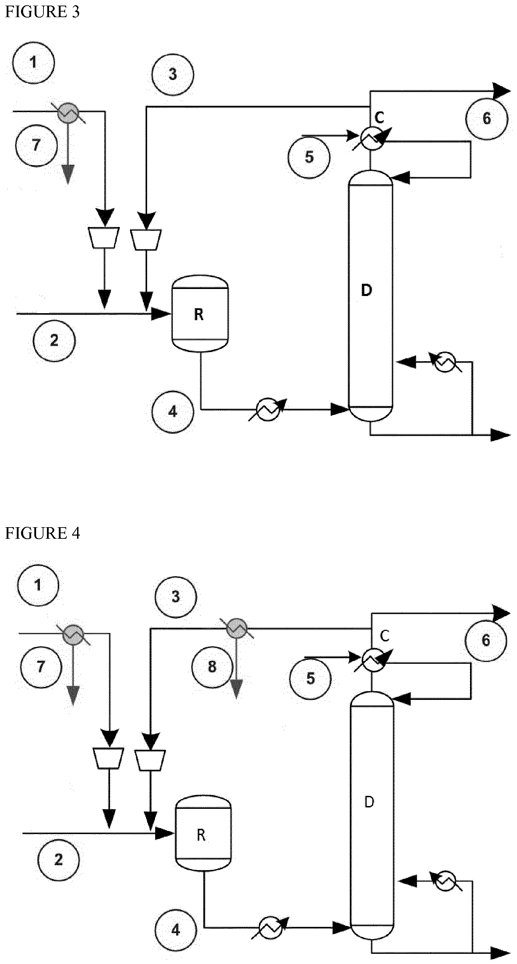

La Figure 3 représente un premier mode de réalisation de l'invention. Par rapport à la Figure 2, un condenseur/refroidisseur a été placé sur le flux d'alimentation d'air 1. Le flux d'eau 7 condensé à 15°C peut être recyclé dans les tours de refroidissement.
Les résultats de simulation Aspen sont rassemblés dans le tableau 2 ci-après.

En éliminant l'humidité présente dans l'air qui alimente le réacteur, on constate que les pertes d'acide acrylique en tête du condenseur sont réduites de près de 50% par rapport au cas de l'exemple 1.

**Tableau 2**

| | |
|---|---|
| AA produit par la réaction, kg/h | 11813 |
| AA perdu en tête du condenseur C, kg/h | 112 |
| Perte d'AA en tête de C, % | 0,95 |
| Température du condenseur de tête C, °C | 58,8 |
| Eau en entrée du réacteur R, % | 5,05 |
| Eau en sortie du réacteur R, kg/h | 6002 |
| AA en sortie du réacteur R, kg/h | 11853 |
| Rapport AA/eau en sortie du réacteur R | 1,97 |

### Exemple 3 (selon l'invention)

La Figure 4 représente un second mode de réalisation de l'invention. Par rapport à la Figure 2, un condenseur/refroidisseur a été placé sur le flux d'alimentation d'air 1, et un condenseur a été placé sur l'effluent 3 recyclé dans le réacteur. Le flux d'eau 7 condensé provenant de l'humidité de l'air peut être recyclé dans les tours de refroidissement. Le condensat 8 est éliminé du procédé.
Selon cette configuration, deux simulations Aspen ont été effectuées, selon le tableau 3.

**Tableau 3**

| | Essai 1 | Essai 2 |
|---|---|---|
| AA produit par la réaction, kg/h | 11818 | 11820 |
| AA perdu en tête du condenseur C, kg/h | 73 | 59 |
| Perte d'AA en tête de C, % | 0,69 | 0,58 |
| Température du condenseur de l'effluent recyclé, °C | 53 | 50 |
| Condensat 8 provenant de l'effluent recyclé, kg/h | 398,4 | 562,9 |
| Condensat recyclé, kg/h | 0 | 0 |
| Perte d'AA dans le condensat, kg/h | 8,3 | 9,5 |
| Température du condenseur de tête C, °C | 57,4 | 56,7 |
| Eau en entrée du réacteur R, % | 4,14 | 3,75 |
| Eau en sortie du réacteur R, kg/h | 5602 | 5430 |
| AA en sortie du réacteur R, kg/h | 11837 | 11833 |
| Rapport AA/eau en sortie du réacteur R | 2,11 | 2,18 |

Dans ce tableau, le pourcentage de perte d'AA en tête du condenseur C tient compte de la perte d'AA présent dans le condensat 8.

Dans ces conditions, même si le flux d'eau condensé à partir de l'effluent gazeux recyclé est éliminé, la perte globale en AA reste inférieure à celle de l'exemple 1 de référence. De plus le condenseur C peut fonctionner à plus basse température.

### Exemple 4 (selon l'invention)

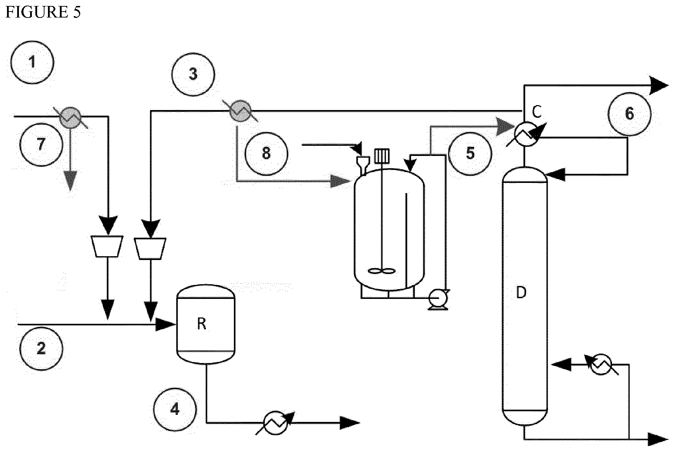

La Figure 5 représente un troisième mode de réalisation de l'invention. Par rapport à la Figure 4, le condensat 8 est introduit dans un réservoir agité, dans lequel est introduit au moins un inhibiteur de polymérisation, et la solution aqueuse d'inhibiteur 5 ainsi préparée in situ peut être directement introduite au niveau du condenseur C.
Les résultats de simulation Aspen sont rassemblés dans le tableau 4 ci-après :

**Tableau 4**

| | |
|---|---|
| AA produit par la réaction, kg/h | 11818 |
| AA perdu en tête du condenseur C, kg/h | 77 |
| Perte d'AA en tête de C, % | 0,65 |
| Température du condenseur de l'effluent recyclé, °C | 53 |
| Condensat 8 provenant de l'effluent recyclé, kg/h | 0 |
| Condensat recyclé, kg/h | 395 |
| Perte d'AA dans le condensat, kg/h | 0 |
| Température du condenseur de tête C, °C | 57,3 |
| Eau en entrée du réacteur R, % | 4,12 |
| Eau en sortie du réacteur R, kg/h | 5596 |
| AA en sortie du réacteur R, kg/h | 11838 |
| Rapport AA/eau en sortie du réacteur R | 2,115 |

Ce mode de réalisation permet de réduire non seulement la perte en AA, mais aussi ne nécessite pas d'eau propre externe pour préparer la solution aqueuse d'inhibiteurs.

## Revendications

1. Procédé de production d'acide (méth)acrylique, comprenant au moins les étapes suivantes :
i) on soumet à une oxydation en phase gazeuse en présence d'air, au moins un précurseur d'acide (méth)acrylique pour former un mélange réactionnel gazeux comprenant de l'acide (méth)acrylique ;
ii) on refroidit le mélange réactionnel gazeux ;
iii) on soumet le mélange réactionnel gazeux à une déshydratation sans utiliser de solvant azéotropique dans une première colonne dite colonne de déshydratation, conduisant à un flux gazeux de tête et à un flux de pied ;
iv) on soumet le flux gazeux distillé en tête de colonne de déshydratation, au moins en partie, à une condensation dans un condenseur de tête, le condensat étant renvoyé à la colonne de déshydratation sous forme de reflux pour absorber l'acide acrylique, et l'effluent gazeux étant renvoyé au moins en partie vers la réaction d'oxydation et le reste étant soumis à une oxydation thermique et/ou catalytique ;
v) on soumet au moins en partie le flux de pied de colonne de déshydratation à une distillation dans une seconde colonne dite colonne de finition, conduisant à un flux de tête, et à un flux de pied contenant des composés lourds ;
vi) on récupère un flux d'acide (méth)acrylique par soutirage latéral de la colonne de finition ;
ledit procédé étant **caractérisé en ce que** l'on met en oeuvre une étape de condensation de l'eau appliquée à l'un au moins des deux flux suivants : l'alimentation en air pour la réaction d'oxydation de l'étape i), ou l'effluent gazeux à la sortie du condenseur de tête de l'étape iv) qui est recyclé à la réaction d'oxydation.

2. Procédé selon la revendication 1 **caractérisé en ce que** l'étape de condensation de l'eau est réalisée à l'aide d'un condenseur unique pour les deux flux ou à l'aide d'un condenseur pour chacun des deux flux.

3. Procédé selon la revendication 1 ou 2 **caractérisé en ce que** l'étape de condensation de l'eau est réalisée à une température allant de 15°C à la température du condenseur de tête de la colonne de déshydratation.

4. Procédé selon l'une quelconque des revendications précédentes **caractérisé en ce que** l'étape de condensation de l'eau pour l'effluent gazeux recyclé est réalisée à une température comprise entre 40°C et 60°C, de préférence entre 45°C et 55°C.

5. Procédé selon l'une quelconque des revendications 1 à 3 **caractérisé en ce que** l'étape de condensation de l'eau pour l'alimentation en air est réalisée à une température comprise entre 15°C et 25°C.

6. Procédé selon l'une quelconque des revendications précédentes **caractérisé en ce que** l'eau condensée issue de l'alimentation en air est recyclée au moins en partie dans les tours de refroidissement d'eau.

7. Procédé selon l'une quelconque des revendications précédentes **caractérisé en ce que** l'eau condensée est utilisée en partie pour préparer des solutions aqueuses d'inhibiteur de polymérisation.

8. Procédé selon l'une quelconque des revendications précédentes **caractérisé en ce que** l'eau condensée est en partie purgée et envoyée au traitement des eaux usées, ou soumise à un traitement d'oxydation thermique.

9. Procédé selon l'une quelconque des revendications précédentes **caractérisé en ce que** le précurseur de l'acide (méth)acrylique est l'acroléine, obtenue par oxydation de propylène ou par oxydéshydrogénation de propane.

10. Procédé selon l'une quelconque des revendications 1 à 8 **caractérisé en ce que** le précurseur de l'acide (méth)acrylique est la méthacroléine obtenue par oxydation d'isobutylène et/ou de tert-butanol ou à partir d'oxydéshydrogénation de butane et/ou isobutane.

11. Procédé selon l'une quelconque des revendications 1 à 8 **caractérisé en ce que** le précurseur de l'acide (méth)acrylique est dérivé du glycérol, de l'acide 3-hydroxypropionique ou de l'acide 2-hydroxypropanoïque.

12. Procédé selon l'une quelconque des revendications 1 à 8 **caractérisé en ce que** l'acide (méth)acrylique est l'acide acrylique et le précurseur de l'acide acrylique est l'acroléine obtenu par oxydation catalytique de propylène.

## Patentansprüche

1. Verfahren zur Herstellung von (Meth)acrylsäure, umfassend mindestens die folgenden Schritte:
i) mindestens ein Vorläufer von (Meth)acrylsäure wird einer Oxidation in gasförmiger Phase im Beisein von Luft unterzogen, um ein gasförmiges Reaktionsgemisch, umfassend (Meth)acrylsäure, zu bilden;
ii) das gasförmige Reaktionsgemisch wird gekühlt;
iii) das gasförmige Reaktionsgemisch wird einer Dehydrierung unterzogen, ohne ein azeotropes Lösungsmittel in einer ersten Säule, Dehydrierungssäule genannt, zu verwenden, was zu einem gasförmigen Kopfstrom und einem Bodenstrom führt;
iv) der destillierte Gasstrom am Kopf einer Dehydrierungssäule wird zumindest teilweise einer Kondensation in einem Kopfkondensator unterzogen, wobei das Kondensat zu der Dehydrierungssäule in Form eines Rückstroms zurückgeschickt wird, um die Acrylsäure zu absorbieren, und wobei der gasförmige Abfluss zumindest teilweise zu der Oxidationsreaktion zurückgeschickt wird, und wobei der Rest einer thermischen und/oder katalytischen Oxidation unterzogen wird;
v) der Bodenstrom einer Dehydrierungssäule wird zumindest teilweise einer Destillation in einer zweiten Säule, Fertigstellungssäule genannt, unterzogen, die zu einem Kopfstrom und einem Bodenstrom, der schwere Verbindungen enthält, führt;
vi) ein Strom von (Meth)acrylsäure wird durch seitliche Extraktion aus der Fertigstellungssäule wiedergewonnen,
wobei das Verfahren **dadurch gekennzeichnet ist, dass** ein Kondensationsschritt des Wassers eingesetzt wird, der an mindestens einem der zwei folgenden Ströme angewandt wird: der Luftversorgung für die Oxidationsreaktion des Schrittes i) oder den gasförmigen Abfluss am Ausgang des Kopfkondensators des Schrittes iv), der zur Oxidationsreaktion rückgeführt wird.

2. Verfahren nach Anspruch 1, **dadurch gekennzeichnet, dass** der Kondensationsschritt des Wassers mit Hilfe eines einzigen Kondensators für beide Ströme oder mit Hilfe eines Kondensators für jeden der zwei Ströme durchgeführt wird.

3. Verfahren nach Anspruch 1 oder 2, **dadurch gekennzeichnet, dass** der Kondensationsschritt des Wassers bei einer Temperatur von 15 °C bis zur Temperatur des Kopfkondensators der Dehydrierungssäule durchgeführt wird.

4. Verfahren nach einem der vorhergehenden Ansprüche, **dadurch gekennzeichnet, dass** der Kondensationsschritt des Wassers für den rückgeführten gasförmigen Abfluss bei einer Temperatur zwischen 40 °C und 60 °C, vorzugsweise zwischen 45 °C und 55 °C, durchgeführt wird.

5. Verfahren nach einem der Ansprüche 1 bis 3, **dadurch gekennzeichnet, dass** der Kondensationsschritt des Wassers für die Luftversorgung bei einer Temperatur zwischen 15 °C und 25 °C durchgeführt wird.

6. Verfahren nach einem der vorhergehenden Ansprüche, **dadurch gekennzeichnet, dass** das kondensierte Wasser aus der Luftversorgung zumindest teilweise in die Wasserkühltürme rückgeführt wird.

7. Verfahren nach einem der vorhergehenden Ansprüche, **dadurch gekennzeichnet, dass** das kondensierte Wasser teilweise verwendet wird, um wässrige polymerisationshemmende Lösungen herzustellen.

8. Verfahren nach einem der vorhergehenden Ansprüche, **dadurch gekennzeichnet, dass** das kondensierte Wasser teilweise gereinigt und zur Abwasseraufbereitung geschickt oder einer thermischen Oxidationsbehandlung unterzogen wird.

9. Verfahren nach einem der vorhergehenden Ansprüche, **dadurch gekennzeichnet, dass** der Vorläufer der (Meth)acrylsäure Acrolein ist, das durch Oxidation von Propylen oder durch Oxydehydrierung von Propan erhalten wird.

10. Verfahren nach einem der Ansprüche 1 bis 8, **dadurch gekennzeichnet, dass** der Vorläufer der (Meth)acrylsäure Methacrolein ist, das durch Oxidation von Isobutylen und/oder Tert-Butanol oder durch Oxydehydrierung von Butan und/oder Isobutan erhalten wird.

11. Verfahren nach einem der Ansprüche 1 bis 8, **dadurch gekennzeichnet, dass** der Vorläufer der (Meth)acrylsäure ein Derivat des Glycerol, der 3-Hydroxypropionsäure oder 2-Hydroxypropansäure ist.

12. Verfahren nach einem der Ansprüche 1 bis 8, **dadurch gekennzeichnet, dass** die (Meth)acrylsäure die Acrylsäure ist, und der Vorläufer der Acrylsäure Acrolein ist, das durch katalytische Oxidation von Propylen erhalten wird.

## Claims

1. (Meth)acrylic acid production process, comprising at least the following steps:
i) at least one (meth)acrylic acid precursor is subjected to gas-phase oxidation in the presence of air so as to form a gas reaction mixture comprising (meth)acrylic acid;
ii) the gas reaction mixture is cooled;
iii) the gas reaction mixture is subjected to dehydration without using azeotropic solvent in a first column, termed dehydration column, resulting in a top gas stream and in a bottom stream;
iv) the gas stream distilled at the top of the dehydration column is at least partly subjected to condensation in a top condenser, the condensate being sent back to the dehydration column in reflux form in order to absorb the acrylic acid, and the gas effluent being at least partly sent back to the oxidation reaction and the remainder being subjected to thermal and/or catalytic oxidation;
v) the stream from the bottom of the dehydration column is at least partly subjected to distillation in a second column, termed finishing column, resulting in a top stream, and in a bottom stream containing heavy compounds;;
vi) a (meth)acrylic acid stream is recovered by drawing off from the side of the finishing column;
said process being **characterized in that** a water condensation step applied to at least one of the following two streams is carried out: the air feed for the oxidation reaction of step i), or the gas effluent at the outlet of the top condenser of step iv) which is recycled to the oxidation reaction.

2. Process according to Claim 1, **characterized in that** the water condensation step is carried out using a single condenser for the two streams or using a condenser for each of the two streams.

3. Process according to Claim 1 or 2, **characterized in that** the water condensation step is carried out at a temperature ranging from 15°C to the temperature of the condenser at the top of the dehydration column.

4. Process according to any one of the preceding claims, **characterized in that** the step of condensing the water for the recycled gas effluent is carried out at a temperature of between 40°C and 60°C, preferably between 45°C and 55°C.

5. Process according to any one of Claims 1 to 3, **characterized in that** the step of condensing the water for the air feed is carried out at a temperature of between 15°C and 25°C.

6. Process according to any one of the preceding claims, **characterized in that** the condensed water from the air feed is at least partly recycled to the water cooling towers.

7. Process according to any one of the preceding claims, **characterized in that** the condensed water is partly used to prepare aqueous solutions of polymerization inhibitors.

8. Process according to any one of the preceding claims, **characterized in that** the condensed water is partly flushed and sent to the treatment of wastewater, or subjected to a thermal oxidation treatment.

9. Process according to any one of the preceding claims, **characterized in that** the (meth)acrylic acid precursor is acrolein, obtained by oxidation of propylene or by oxydehydrogenation of propane.

10. Process according to any one of Claims 1 to 8, **characterized in that** the (meth)acrylic acid precursor is methacrolein obtained by oxidation of isobutylene and/or of tert-butanol or from oxydehydrogenation of butane and/or isobutane.

11. Process according to any one of Claims 1 to 8, **characterized in that** the (meth)acrylic acid precursor is derived from glycerol, from 3-hydroxypropionic acid or from 2-hydroxypropanoic acid.

12. Process according to any one of Claims 1 to 8, **characterized in that** the (meth)acrylic acid is acrylic acid and the acrylic acid precursor is acrolein obtained by catalytic oxidation of propylene.
